# EUROPÄISCHE PATENTSCHRIFT

(11) **EP 1 748 702 B1**
(45) Veröffentlichungstag und Bekanntmachung des Hinweises auf die Patenterteilung: **14.05.2008**
(21) Anmeldenummer: 05741371.8
(22) Anmeldetag: 06.05.2005
(51) Int. Cl.: A23K 1/16

(54) **ALKALOIDHALTIGES FUTTERMITTEL BZW. FUTTERMITTELZUSATZ**
FEED OR FEED ADDITIVE CONTAINING AN ALKALOID
ALIMENT POUR ANIMAUX CONTENANT UN ALCALOIDE ET COMPLEMENT POUR ALIMENTS POUR ANIMAUX

(30) Priorität: 19.05.2004 AT 8822004
(43) Veröffentlichungstag der Anmeldung: 07.02.2007
(73) Patentinhaber: Roth, Hermann, 65343 Eltville (DE)
(72) Erfinder: Roth, Hermann, 65343 Eltville (DE)
(74) Vertreter: Puchberger, Peter
(86) Internationale Anmeldenummer: PCT/EP2005/004946
(87) Internationale Veröffentlichungsnummer: WO 2005/115165

(56) Entgegenhaltungen:
- WO-A-02/21933
- DE-A1- 4 303 099

## Beschreibung

Diese Erfindung betrifft ein Futtermittel zur Appetit- und Leistungsförderung für Nutztiere, wobei herkömmliche Futterstoffe, wie Getreide oder Getreideprodukte, Mais, Proteine und aromatische Aminosäuren, Vitamine, mineralische Zuschlagstoffe, wie Salze, Phosphate, Kalk, Enzyme und dergleichen enthalten sind, sowie Futtermittelzusatz zur Herstellung derartiger Futtermittel.

So genannte Leistungsförderer kommen bei der Nutztierfütterung häufig zum Einsatz. Es handelt sich dabei um Substanzen, welche die Nährstoffaufnahme in Pansen oder Darm optimieren. Dadurch wird die Futterverwertung verbessert und der Futterverbrauch pro Kilogramm Gewichtszunahme reduziert. Man unterscheidet antibiotische, probiotische und chemische Leistungsförderer.

In letzter Zeit wird jedoch die Verwendung von Leistungsförderern, insbesondere von antibiotischen und chemischen Leistungsförderern, von den Konsumenten, aber auch von manchen Fachleuten immer mehr abgelehnt, da einerseits eine Beeinträchtigung der menschlichen Gesundheit durch Rückstände der verwendeten Substanzen in tierischen Nahrungsmitteln, andererseits die Resistenzbildung gegen Mikroorganismen, insbesondere auch humanpathogene Mikroorganismen, befürchtet wird. Viele der als Leistungsförderer verwendeten Stoffe sind mittlerweile auch in verschiedenen Ländern als Futteradditive verboten.

Andererseits ist festzuhalten, dass der Einsatz von Leistungsförderern von vielen Tierhaltern befürwortet wird, da diese Mittel nicht nur die tierische Wachstumsleistung fördern, sondern auch den Gesundheitsstatus der Tiere verbessern. Somit ist die Suche nach geeigneten Ersatzstoffen, die als alternative Leistungsförderer die konventionellen ersetzen sollen, in den Mittelpunkt des Interesses gerückt.

Aus der DE 43 03 099 ist die Verwendung von Benzophenanthridinalkaloiden zur Leistungsförderung bekannt. Diese Alkaloide sind insbesondere in der *Sanguinaria canadensis* enthalten, die jedoch als natürliches Pflanzenmaterial nur in beschränktem Umfang zur Verfügung steht, so dass diese Alkaloide relativ teuer sind.

Aus der WO 02/21933 ist die Verwendung eines Protoberberinalkaloids in Kombination mit einem Benzophenanthridinalkaloid als Leistungs- oder Appetitförderer für Nutztiere bekannt geworden.

Überraschend hat sich nun gezeigt, dass die Appetit- und Leistungsförderung durch die Verwendung von Protopinalkaloiden, insbesondere α-Allocryptopin, signifikant verbessert werden kann.

Der Einsatz von α-Allocryptopin in der Tierhaltung ist bisher nicht bekannt.

Gemäß einem Merkmal der Erfindung ist ein Futtermittel der eingangs genannten Art oder ein Futtermittelzusatz bzw. Futterzusatzstoff(e) zur Herstellung eines solchen Futtermittels vorgesehen, das/der ein Protopinalkaloid, insbesondere α-Allocryptopin, in wirksamer Menge als Leistungs- und Appetitförderer für Nutztiere enthält.

Gemäß einem weiteren Merkmal der Erfindung kann das Futtermittel oder der Futtermittelzusatz ein Protopinalkaloid, insbesondere α-Allocryptopin, in Kombination mit mindestens einem Benzophenanthridinalkaloid enthalten. Durch die Verwendung einer solchen Alkaloidkombination kann ein unerwarteter Synergieeffekt erreicht werden.

Von α-Allocryptopin wird angenommen, dass es eine hepatoprotektive Substanz ist. Es gibt entsprechende Literatur über seine Anwendung in der Humanmedizin. Eine Kombination mit den Benzophenanthridinalkaloiden scheint überlegenswert, da die Wirkungssysteme synergistisch sein sollten. Benzophenanthridinalkaloide wirken geringgradig antimikrobiell und haben eine schützende Wirkung auf essentielle Nährstoffe wie Tryptophan, Lysin etc. α-Allocryptopin schützt die Leber und hat einen vermuteten regenerativen Nutzen, der besonders bei wachsenden Tieren, in der Laktation und bei Geflügel wertvoll sein sollte, da man dort häufig klinische Leberprobleme diagnostiziert (Schweine, Sauen, Milchkuh; Mastrind) oder mit Fettlebersyndrom große wirtschaftliche und gesundheitliche Probleme als Standard auftreten (Mastgeflügel, Legehennen).

Gemäß einem Aspekt der vorliegenden Erfindung können beide Effekte kombiniert werden, da eine bessere Versorgung mit essentiellen Aminosäuren dem Entgiftungssystem der Leber das notwendige Substrat bietet sowie dann durch die Kombination mit α-Allocryptopin dieser Prozess und der gesamte Leberstoffwechsel stark angeregt wird. Die beschriebenen Prozesse können letztlich zu einer synergistischen Symbiose führen, die sich durch höhere Leistungswerte, bessere Gesundheit, höhere Langlebigkeit, reduzierten Einsatz von Medikamenten und eine abgesicherte Umwelt-, Öko- und Betriebsbilanz auszeichnet.

Gemäß diesem Merkmal kann die Erfindung somit über die eigenständigen Effekte der jeweils einzeln eingesetzten Substanzen Benzophenanthridinalkaloid oder α-Allocryptopin hinausgehen und einen enormen Fortschritt für die Produktion und den Verbraucher darstellen.

Gemäß einem weiteren Merkmal der Erfindung kann das Protopinalkaloid und/oder die Benzophenanthridinalkaloide in Form von Pflanzenmaterial, als Pflanzenpresssaft oder in Form von Extrakten aus Pflanzenmaterial zum Einsatz kommen. Beispielsweise kann das α-Allocryptopin in Form von Pflanzenmaterial der *Macleaya cordata* oder als Extrakt davon verwendet werden. In dieser Erfindung verwendbare Extrakte können nach jedwedem bekannten Verfahren hergestellt werden, und es können beispielsweise wässrige und/oder alkoholische Extrakte und/oder CO₂-Extrakte eingesetzt werden.

Selbstverständlich können das verwendete Protopinalkaloid ebenso wie das/die Benzophenanthridinalkaloid(e) sowohl als isolierte Alkaloide bzw. Alkaloidgemische als auch in Form ihrer Derivate oder synthetischen Analoge zum Einsatz kommen.

Es können auch beliebige Mischungen von Pflanzenmaterial, Pflanzenpresssaft, Extrakten aus Pflanzenmaterial, isolierten Alkaloiden, deren Derivaten und deren synthetischen Analogen verwendet werden.

Die im Futter enthaltene Alkaloidmenge ist nach unten lediglich durch die Wirksamkeit begrenzt. Die Gesamtalkaloidmenge pro Tonne Futtermittel liegt bevorzugt im Bereich von 1 mg bis 100 g.

Die Vorteile des erfindungsgemäß hergestellten Futtermittels oder Futtermittelzusatzes, das/der Protopinalkaloide, vorzugsweise α-Allocryptopin, insbesondere in Kombination mit Benzophenanthridinalkaloiden, aufweist, werden durch folgende Beobachtungen belegt:
◆ Die Leber von Hobby- und Nutztieren, denen dieses Futter verabreicht wird; ist deutlich gesünder als bei Tieren der jeweiligen Kontrollgruppen. Dies zeigt sich an der dunkler roten Färbung, die auf geringere Fetteinlagerungen schließen lässt.
◆ Die Leber der Versuchstiere hat ein geringeres Gewicht in % der Körpermasse, da weniger Fett und anderes Gewebe eingelagert wird. Dies ist positiv zu bewerten, da es auf weniger "Leberstress" hinweist.
◆ Die Versuchstiere zeigen deutlich höhere Futteraufnahme als die der Kontrollgruppen, wobei einerseits anzumerken ist, dass dieser Effekt wesentlich über das Niveau des Effektes von Aromastoffen hinausgeht, und andererseits davon auszugehen ist, dass diese vorteilhafte Wirkung auf bessere Gesundheit des Verdauungsapparats der Versuchstiere, insbesondere verbesserte Gesundheit der Leber, zurückzuführen ist.
◆ Aufgrund der besseren Futteraufnahme zeigt sich insgesamt verbesserte Leistung der Versuchstiere sowie eine günstigere Abwehrlage gegen Stress und Krankheiten aufgrund der verbesserten Lebergesundheit.
◆ Der Tryptophan-Gehalt des Pfortaderblutes der Versuchstiere ist deutlich verbessert gegenüber jenem der Kontrolltiere, was wiederum auf eine hepatoprotektive Wirkung und somit auf verbesserte Lebergesundheit hinweist.

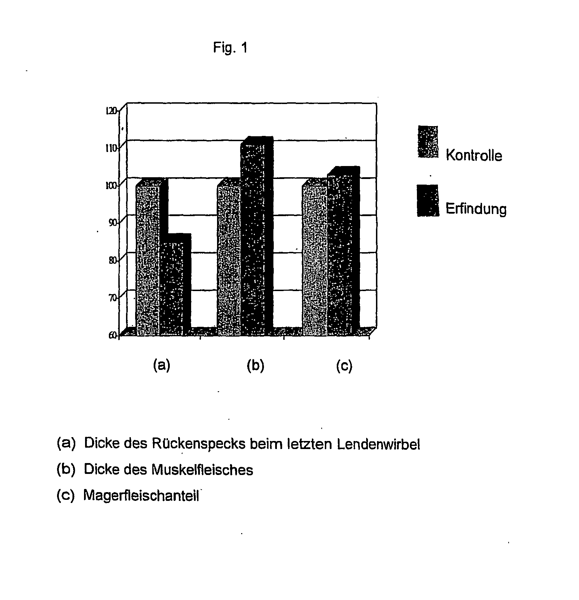

Die Vorteile der vorliegenden Erfindung werden im Folgenden anhand der Beispiele und der beigefügten Zeichnung erläutert, wobei die Fig. 1 eine graphische Darstellung der mit Hilfe eines alkaloidhaltigen Futtermittels gemäß dieser Erfindung erzielten Effekte auf die Leistungs- und Schlachtkörper-Parameter bei Mastschweinen ist.

### Beispiel 1:

Pelletierte Pflanzenteile der *Macleaya cordata* wurden gepulvert und mit angesäuertem Methanol (0,1 % HCl) 12 Stunden in einem Soxhlet-Extraktor extrahiert.

Der Extrakt wurde mittels HPLC analysiert. Die HPLC-Analyse wurde auf einem mit einem UV-Detektor SPD-10Avp und einem fluorimetrischen Detektor RF-10Axl ausgestatteten Shimadzu Class VP Gerät unter Verwendung einer Purospher^{®} Star RP-18e Umkehrphasen-Säule durchgeführt. Die mobile Phase war 1-Heptansulfonsäure/Triethylamin in 25 % Acetonitril in einem Gradienten mit 40 % Acetonitril. Die Detektion erfolgte mittels UV bei 285 nm und/oder durch Fluorimetrie bei 327 nm Anregung / 577 nm Emission. Referenzlösungen von Alkaloiden wurden in der mobilen Phase als externer Standard verwendet. Alle Analysen wurden dreifach durchgeführt. Die Analysenergebnisse bezüglich der Leitalkaloide sind in der Tabelle 1 angeführt.

### Beispiel 2:

Ein durch ethanolische wässrige Extraktion hergestellter *Macleaya-cordata-*Extrakt wurde in einer Konzentration von 1 mg/ml in Methanol gelöst. Die Lösung wurde einer HPLC-Analyse unterworfen. Vor der Analyse wurde sie mit der mobilen Phase verdünnt. Die HPLC-Analyse erfolgte wie im Beispiel 1 beschrieben. Die Analysenergebnisse bezüglich der Leitalkaloide sind in der Tabelle 1 angeführt.

### Beispiel 3:

Ein weiterer durch ethanolische wässrige Extraktion hergestellter *Macleaya-cordata-*Extrakt wurde in einer Konzentration von 1 mg/ml in Methanol gelöst. Die Lösung wurde einer HPLC-Analyse unterworfen. Vor der Analyse wurde sie mit der mobilen Phase verdünnt. Die HPLC-Analyse erfolgte wie im Beispiel 1 beschrieben. Die Analysenergebnisse bezüglich der Leitalkaloide sind in der Tabelle 1 angeführt.

### Beispiel 4:

In diesem Beispiel wurde ein Futtermitteladditiv analysiert, das aus 95 % getrockneten *Macleaya-cordata*-Pflanzenteilen und 5 % Extrakt aus *Macleaya cordata* bestand. Die Probe wurde mit angesäuertem Methanol (0,1% HCl) 12 Stunden in einem Soxhlet-Extraktor extrahiert. Der Extrakt wurde mittels HPLC untersucht, wie im Beispiel 1 beschrieben. Die Analysenergebnisse bezüglich der Leitalkaloide sind ebenfalls in der Tabelle 1 angeführt.

**Tabelle 1**

| | *Macleaya cordata* Pflanzenteile | *Macleaya cordata* Extrakt | *Macleya cordata* Extrakt | Futtermitteladditiv |
|---|---|---|---|---|
| | (Beispiel 1) (mcg/g) | (Beispiel 2) (mg/g) | (Beispiel 3) (mg/g) | (Beispiel 4) (mg/g) |
| α-Allocryptopin | 6,8 ± 0,3 | 21 ± 4 | 6 ± 3 | 15,33 |
| Sanguinarin | 6,5 ± 0,3 | 402 ± 19 | 213 ± 9 | 16,5 |
| Chelerythrin | 4,7 ± 0,3 | 125 ±7 | 102 ± 6 | 9,33 |

### Beispiel 5:

Mastferkel wurden in zwei Stallabteilen in gleichgeschlechtlichen Zweiergruppen untergebracht. Die Tiere wurden in zwei Versuchsgruppen untergeteilt.

Die Ferkel erhielten ein herkömmliches Futter aus Weizen, Gerste, HP Sojaschrot, Mineralstoffen, Spurenelementen, Vitaminen und Aminosäuren, wobei dem Futter der Versuchsgruppe 1 Benzophenanthridinalkaloide und dem Futter der Versuchsgruppe 2 α-Allocryptopin in Kombination mit Benzophenanthridinalkaloiden zugesetzt waren. Die Dosierung der Wirkstoffe ist in der Tabelle 2 angegeben. Das Additiv, das dem Futter der Versuchsgruppe 2 zugesetzt wurde, enthielt Benzophenanthridinalkaloide und α-Allocryptopin im Verhältnis von etwa 3:1.

**Tabelle 2**

| Versuchsgruppe | Zugesetzte Alkaloide | Dosierung der Wirkstoffe |
|---|---|---|
| 1 | Benzophenanthridinalkaloide | 0,5 mg/kg |
| 2 | α-Allocryptopin + Benzophenanthridinalkaloide | 0,8 mg/kg |

Die Tiere wurden am Beginn und am Ende des Versuchs wöchentlich sowie während der Versuchsdauer 14-tägig gewogen. Drei der Tiere fielen während der Mastperiode aus. Die Ergebnisse des Mastversuchs von 30 bis 105 kg Lebendgewicht sind in der Tabelle 3 angeführt.

**Tabelle 3**

| Versuchsgruppe | 1 | 2 |
|---|---|---|
| Alkaloide im Futter | Benzophenanthridinalkaloide | α-Allocryptopin + Benzophenanthridinalkaloide |
| Mastdauer, Tage | 86,3 | 81,6 |
| Futteraufnahme, kg/Tag | 2,09 | 2,21 |
| Zunahme, g/Tag | 874 | 931 |
| Futterverwertung, kg | 2,39 | 2,38 |
| Fleischanteil, % | 59,9 | 59,3 |
| Fleischbeschaffenheitszahl, Punkte | 53,5 | 54,3 |

Wie man anhand der Tabelle 3 sieht, ist das Mastleistungsniveau der Tiere sehr hoch. Die Versuchsgruppe 2 zeigte die höhere Futteraufnahme. Die Versuchsgruppe 1 zeigte eine geringere Zunahme pro Tag, und zwar 57 g weniger als die Versuchsgruppe 2. Diese 57 g entsprechen bei einer Mast von 30 kg auf 105 kg etwa 5 Masttagen.

Die mit einem erfindungsgemäßen Futtermittel gefütterte Versuchsgruppe 2 zeigte die höheren Futteraufnahmen und Tageszunahmen. Dies ist ein Hinweis auf hohen Appetit, der auf eine verbesserte Lebergesundheit zurückgeführt wird. Auch die Fleischbeschaffenheitszahl ist mit 54,3 bei dieser Versuchsgruppe höher und weist ebenfalls auf eine effizientere Proteinsynthese der Leber hin.

### Beispiel 6:

In zwei Versuchsdurchgängen wurde an jeweils 12 x 7 männlichen Broilern (Ross 308) der Einfluss von 25, 50 bzw. 100 ppm alkaloidhaltigem Futtermitteladditiv untersucht. Das Futtermittel war ein handelsübliches Futter für die Broilermast, dem 0, 25, 50. bzw. 100 ppm alkaloidhaltiges Additiv zugesetzt wurden. Dieses Additiv bestand aus einer Mischung von gemahlenen Rhizomen der *Sanguinaria canandensis* und Pflanzenteilen der *Macleaya cordata* und enthielt etwa 1,5 % Sanguinarin, 0,8 % Chelerythrin und 0,35 % α-Allocryptopin. Analysendaten des erfindungsgemäßen alkaloidhaltigen Futtermittels sind in der Tabelle 4 angeführt.

**Tabelle 4: Analysendaten des erfindungsgemäßen alkaloidhaltigen Futtermittels sowie des Kontroll-Futtermittels**

| Gehalt an Additiv | | 0 ppm | 25 ppm | 50 ppm | 100 ppm |
|---|---|---|---|---|---|
| α-Allocryptopin (ppb) | | 0 | 230 | 465 | 856 |
| Benzophenanthridinalkaloide (ppb) | | 0 | 1013 ± 25 | 1963 ± 60 | 3850 ± 85 |
| Trockensubstanz (%) | | 87,9 ± 0,6 | 88,1 ± 0.7 | 88,0 ± 1,0 | 88,3 ± 0,3 |
| Gehalt pro kg (bei 890 g Trockensubstanz) | | | | | |
| | Asche (g) | 60 ±1,2 | 60 ± 1.9 | 59 ± 2.0 | 60 ± 1,9 |
| | Rohprotein (g) | 199 ± 3,2 | 200 ± 3,6 | 200 ± 1.0 | 200 ± 5.1 |
| | Fett (Soxhlet) (g) | 63 ± 1,5 | 64 ± 2,4 | 63 ± 1,5 | 62 ± 1,7 |
| | Faser (g) | 28 ± 2,1 | 26 ± 2,0 | 27 ± 0,7 | 27 ± 1,0 |
| Gesamtenergie (MJ) | | 17,4 ± 0,2 | 17,4 ± 0,1 | 17,4 ± 0,2 | 17,4 ± 0,1 |

Die Tabelle 5 zeigt die Wachstums- und Schlachtkörperparameter.

**Tabelle 5**

| | | | | | | | | |
|---|---|---|---|---|---|---|---|---|
| Gehalt an Additiv | 0 ppm | | 25 ppm | | 50 ppm | | 100 ppm | |
| | Ø | rel⁽¹⁾ | Ø | rel | Ø | rel | Ø | rel |
| Körpergewicht ( = BM) am Tag 1 (g) | 46 | 100 | 47 | 101 | 46 | 99,1 | 46 | 100 |
| BM am Tag 40 (g) | 2488 | 100 | 2464 | 99,0 | 2526 | 101,5 | 2474 | 99,5 |
| tägl. Gewichtszunahme (g) | 62,6 | 100 | 62,0 | 99,0 | 63,6 | 101,6 | 62,3 | 99,5 |
| Futter pro Tag (g) | 103 | 100 | 99 | 96,3 | 102 | 99,5 | 102 | 99,6 |
| Futter pro kg BM (kg) | 1,64 | 100 | 1,60 | 97,6 | 1,61 | 98,2 | 1,64 | 100 |
| Schlachtgewicht (g) | 1799 | 100 | 1774 | 98,6 | 1834 | 101,9 | 1766 | 98,1 |
| Karkassenausbeute (%) | 72,1 | 100 | 72,0 | 99,9 | 72,6 | 100,7 | 71,4 | 99,0 |
| Farbe der Leber⁽²⁾ | 1,19 | 100 | 1,06 | 89 | 1,05 | 88 | 1,07 | 90 |
| Wasser pro Tag (ml) | 202 | 100 | 202 | 100 | 197 | 97,4 | 196 | 97,2 |
| Wasser: Futter (ml/g) | 1,97 | 100 | 2,05 | 104 | 1,92 | 97,5 | 1,92 | 97,5 |

| | | | | | | | | |
|---|---|---|---|---|---|---|---|---|
| (1) Ø= Durchschnitt, rel = Relativwert (in %) (2) 1 = dunkelrot (gesund), 2 = gelblich (krank; Fettleber, Hepatitis) | | | | | | | | |

Die in der Tabelle 5 angeführten Parameter zeigen, dass das Schachtgewicht beim Vergleich mit der Kontrollgruppe bei der Versuchsgruppe, der ein Futtermittel mit 50 ppm alkaloidhaltigem Additiv verabreicht wurden, am höchsten war. Die Futterverwertung war in den Versuchsgruppen, denen Futtermittel mit 25 bzw. 50 ppm Additiv verabreicht wurden, verbessert. Diese Ergebnisse waren statistisch nicht signifikant, da nur vier Gruppen zur Verfügung standen und der Versuch bei einem sehr hohen Leistungsniveau stattfand. Bei einer höheren Gruppenanzahl von beispielsweise 10 Wiederholungen wäre das Ergebnis statistisch abzusichern gewesen.

In allen Versuchsgruppen hatten die Tiere eine dunklere Leber als die Broiler der Kontrollgruppe, was ein deutlicher Hinweis auf die hepatoprotektive Wirkung des erfindungsgemäßen Futtermittels ist.

Im Rahmen dieser Versuchsreihe wurden auch die Energieverwertung, die Stickstoffeinlagerung sowie der Trockensubstanzgehalt der Exkremente untersucht. Die Ergebnisse sind in der Tabelle 6 angeführt.

**Tabelle 6**

| Gehalt an Additiv | 0 ppm | | 25 ppm | | 50 ppm | | 100 ppm | |
|---|---|---|---|---|---|---|---|---|
| | Ø | rel⁽¹⁾ | Ø | rel | Ø | rel | Ø | rel |
| Energieverwertung | 0,77 | 100 | 0,78 | 101 | 0,78 | 101 | 0,77 | 100 |
| Stickstoffeinlagerung | 0,60 | 100 | 0,62 | 103 | 0,61 | 102 | 0,60 | 100 |
| Trockensubstanzgehalt der Exkremente (%) | 28,9 | 100 | 29,2 | 101 | 29,0 | 100 | 29,3 | 101 |

| | | | | | | | | |
|---|---|---|---|---|---|---|---|---|
| ⁽¹⁾Ø = Durchschnitt, rel = Relativwert (in %) | | | | | | | | |

Die Versuchsgruppen, deren Futter 25 bzw. 50 ppm alkaloidhaltiges Additiv zugesetzt wurde, zeigten tendenziell bessere Energieverwertung und Stickstoffeintagerung. Der gemessene Energiegehalt des Futters, 17,4 MJ/kg, zusammen mit der Energieverwertung von 0,77 bis 0,78 führt zu einem Gehalt an metabolisierbarer Energie im Futter von 13,43 bis 13,62 MJ/kg. Die Exkremente aller Versuchsgruppen zeigten einen hohen Gehalt an Trockensubstanz, wobei es kaum Unterschiede zwischen den Versuchsgruppen gab.

### Beispiel 7: Der Einfluss von α-Allocryptopin und Benzophenanthridinalkaloiden auf ausgewählte Parameter der Eiweißverwertung unter standardisierter Fütterung

Dieses Beispiel zeigt den Einfluss von α-Allocryptopin und Benzophenanthridinalkaloide enthaltendem Futter auf Parameter des Proteinstoffwechsels. Es wird gezeigt, dass die Erfindung durch eine irreversible Hemmung der AAD (Aromatische Aminosäure-Decarboxylase) durch Benzophenanthridinalkaloide *in vivo* stattfindet und so die Versorgung der Tiere mit essentiellen Nährstoffen wie Tryptophan und Phenylalanin, aber auch anderen essentiellen Aminosäuren verbessert ist oder Einsparmöglichkeiten im Einsatz dieser Nährstoffe in kommerziellem Futter durch die Verwendung dieser Alkaloide entstehen, die so bisher nicht bekannt und nutzbar waren.

Zwei Gruppen mit 6 kastrierten Schweinen wurden in Koben gehalten. Am Beginn des Versuchs (Tag 0) betrug das mittlere Körpergewicht der Tiere der Kontrollgruppe 36,08 ± 3,79 kg und das der Tiere der Versuchsgruppe 36,02 ± 3,74 kg. Die Tiere beider Gruppen wurden dreimal am Tag mit dem in der Tabelle 7 angegebenen Futter gefüttert, wobei jedoch das Futter der Versuchsgruppe α-Allocryptopin und Benzophenanthridinalkaloide, insbesondere Sanguinarin und Chelerythrin, enthielt, und zwar in Form von 30 ppm eines Additivs aus Pflanzenmaterial der *Sanguinaria canadensis* und der *Macleaya cordata.* Der Wirkstoffgehalt war 2,2 % Benzophenanthridinalkaloide und 0,1 % α-Allocryptopin.

**Tabelle 7: Zusammensetzung des Futters und Nährwert**

| | | | |
|---|---|---|---|
| Gerste | 64,0 % | Rohprotein | 16,5 % |
| Sojaschrot | 18,9% | metabolisierbare Energie | 14,0 MJ/kg |
| Sojaöl | 7.9% | Rohfaser | 5,0 % |
| Weizenkleie | 6,4% | Rohasche | 5,5 % |
| Andere Bestandteile | 2,8% | Rohfett | 9,8% |
| Gesamt | 100,0 % | Lysin | 0,9% |
| | | Methionin/Cystein | 0,5 % |
| | | Threonin | 0,6 % |
| | | Tryptophan | 0,2 % |
| | | Phosphor | 0,6 % |
| | | Natrium | 0,1 % |
| | | Calcium | 0,9 % |

Die Tiere hatten freien Zugang zu Trinkwasser, aber während der Adaptierungsphase (Tag 33-39), in der die Tiere einzeln in einem Stoffwechselkäfig gehalten wurden, und während der anschließend durchgeführten N-Bilanz-Versuche (Tag 40 - 49) wurde nur nach der Fütterung Wasser bis zur Sättigung gegeben.

Urinproben wurden einmal am Tag, Kotproben dreimal am Tag genommen. Am Tag 42 und 49 wurden aus der Ohrvene Blutproben für die Bestimmung des Ammonium- und des Harnstoffspiegels entnommen.

Am Ende des Versuchs war das mittlere Körpergewicht der Tiere der Versuchsgruppe etwas höher (76,27 ± 5,11 kg) als das der Tiere der Kontrollgruppe (75,09 ± 7,42 kg). Ähnliches wurde bei der täglichen Gewichtszunahme beobachtet. Sie betrug bei der Versuchsgruppe 821,3 ± 44.7 g und bei der Kontrollgruppe 796,1 ± 86,2 g. Dabei war die tägliche Futteraufnahme bei beiden Gruppen durch rationierte Fütterung gleich, was das Versuchsziel war: Versuchsgruppe: 1775,9 ± 91,5 g; Kontrollgruppe 1770,8 ± 120,3 g. Die Futterverwertung war bei der Versuchsgruppe mit 2,196 ± 0,094 besser als bei der Kontrollgruppe 2,237 ± 0,173.

Die N-Bilanz-Studie, erstellt nach wissenschaftlichen Grundsätzen auf Basis eines korrigierten und somit vergleichbaren metabolischen Körpergewichts (Körpermasse^{0,75} entspricht der sogenannten metabolischen Körpermasse, wodurch der Stoffwechsel eines Elefanten mit dem einer Maus auf wissenschaftlicher Grundlage vergleichbar gemacht wird), zeigte, dass die N-Aufnähme bei der Kontrollgruppe (2,057 ± 0,011 g/W^{0,75}/Tag) und bei der Versuchsgruppe (2,062 ± 0,010 g/W^{0,75}/Tag) auf gleichem Niveau war, was durch die rationierte Futtergabe zwingend gegeben war. Auch die Messung der N-Ausscheidung in den Faeces ergab für die Kontroll- und die Versuchsgruppe fast gleich Werte, nämlich 0,360 ± 0,023 g/W^{0,75}/Tag bzw. 0,370 ± 0,044 g/W^{0,7}/Tag.

Bei der N-Ausscheidung im Urin fand man jedoch deutliche Unterschiede, nämlich 0,774 ± 0,094 g/W^{0,75}/Tag bei der Versuchsgruppe im Vergleich zu 0,860 ± 0,135 g/W^{0,75}/Tag bei der Kontrollgruppe. Dies zeigt, dass bei der Versuchsgruppe der N-Verlust deutlich um 11 % reduziert war. Diese Daten belegen, dass das Mittel erfindungsgemäß zu einer deutlich verbesserten Ausnutzung des gesamten gefütterten Proteins aus dem Futter führt, was Gegenstand der Erfindung ist und hohen wirtschaftlichen und umweltrelevanten Nutzen hat.

Diese Daten werden gestützt durch die N-Retention, d.h den Proteinansatz im Tierkörper, der bei der Kontrollgruppe 0,837 ± 0,133 g/W^{0,75}/Tag und bei der Versuchsgruppe 0,918 ± 0,084 g/W^{0,75}/Tag betrug, womit die Erfindung zu einer um 10 % erhöhten N-Retention führt, was gleichzusetzen ist mit einer um 10 % verbesserten Ausnutzung des Proteins aus dem Futter und den Eiweißpflanzen, wodurch die Nahrungsqualität verbessert und die Stickstoffbelastung des Grundwassers um besagte 10 % reduziert wird.

Die scheinbare N-Verdaulichkeit scheint durch das α-Allocryptopin und Benzophenanthridinalkaloide enthaltende Futtermittel nicht beeinflusst zu werden. Die berechneten Prozentsätze waren 82,53 ± 1,10 % bei der Kontrollgruppe und 82,07 ± 2,06 % bei der Versuchsgruppe. Die Daten zur N-Verwertung zeigen jedoch deutlich um 10 % erhöhte Werte bei der Versuchsgruppe (44,52 ± 4,24 %) im Vergleich zur Kontrollgruppe (40,73 ± 6,63), womit die *in vivo* intermediär stattfindende Wirkung von α-Allocryptopin und den Benzophenanthridinalkaloiden auf den Proteinansatz und die Proteinausnutzung im Organismus des Tieres belegt sind.

Wie man der nachstehenden Tabelle 8 entnehmen kann, hatte der Alkaloidgehalt des Futtermittels keinen Einfluss auf die Entwicklung des Ammoniumgehalts in den Blutproben, die an zwei Tagen im Abstand von einer Woche während der Phase der N-Bilanz-Versuche genommen wurden, sie ergab aber deutlich niedrigere Harnstoff-Spiegel im Blut der Versuchstiere. Niedrige Harnstoffwerte sind ein deutliches Zeichen für eine bessere Ausnutzung des absorbierten Eiweißes aus dem Futter und für geringeren Aufwand der Leber für Entgiftung des giftigen Stickstoffs aus nicht genutztem Futtereiweiß. Dieser deutliche Effekt wurde mit alleiniger Gabe von Benzophenanthridinalkaloidennicht erreicht, sondern ergab sich aus deren Kombination mit dem Alkaloid α-Allocryptopin. Eine höhere Tierzahl je Gruppe hätte hier zu signifikanten Ergebnissen geführt.

**Tabelle 8: Einfluss des alkaloidhaltigen Futtermittels auf Ammonium- und Harnstoff-Spiegel im Blut (n = 6)**

| | Ammonium [µmol/ml] | | Harnstoff [mmol/ml] | |
|---|---|---|---|---|
| | Tag 42 | Tag 49 | Tag 42 | Tag 49 |
| Kontrollgruppe | 48,33 ± 5.05 | 39,17 ± 7,98 | 3,87 ± 0,74 | 3,88 ± 0,99 |
| Versuchsgruppe | 41,17 ± 3.76 | 39,17 ± 3,97 | 2,92 ± 0,36 | 3,00 ± 0,24 |

Es zeigt sich, dass der Zusatz von α-Allocryptopin und Benzophenanthridinalkaloiden zum Futter deutlich positive Effekte in Bezug auf die untersuchten Leistungsparameter zeigte, wobei die Auswirkung auf die Futterverwertung am stärksten war. Es zeigen sich deutliche Unterschiede in Bezug auf den N-Bilanz-Versuch sowie die Ammonium- und Harnstoffspiegel im Blut, die jedoch aufgrund der geringen Anzahl von Versuchstieren statistisch nicht absicherbar waren. Die reduzierten Werte an Ammonium und Harnstoff im Blut sind ein Beleg dafür, dass die Tiere das aufgenommene Eiweiß besser nutzten, und somit für die bessere Lebertätigkeit. Die beobachtete Verschiebung hin zu einer Stickstoff-Ersparnis, die ein Hinweis auf die Auswirkung auf den Proteinstoffwechsel ist, kann bestätigen, dass die Hemmung der AAD durch Benzophenanthridinalkaloide *in vivo* stattfindet und wirtschaftlich hohen Nutzen hat.

### Beispiel 8: Der Einfluss von α-Allocryptopin und Benzophenanthridinalkaloiden im Futtermittel auf die Futteraufnahme bei Mastschweinen bei unbeschränktem Zugang zu Futter (ad-libitum-Fütterung)

12 kastrierte Schweine wurden in Zweiergruppen mit separaten Futtertrögen gehalten. Der Kontrollgruppe wurde das in der Tabelle 9 angeführte Futter verabreicht. Die Versuchsgruppe erhielt dieses Futter versetzt mit 30 ppm des im Beispiel 7 genannten Additivs, bestehend aus Sanguinarin, Chelerythrin und α-Allocryptopin.

**Tabelle 9: Zusammensetzung des Futters (metabolisierbare Energie: 13,5 MJ/kg; 18 % Rohprotein)**

| Bestandteil | % |
|---|---|
| Mais | 51 |
| Gerste | 21 |
| Sojaschrot | 11 |
| Fischmehl | 8 |
| Maiskleber | 6 |

| Vitamin/Mineral-Prämix | 3 |
|---|---|
| Lysin | 0,24 |
| Threonin | 0,18 |
| Tryptophan | 0,07 |
| Methionin | 0,07 |

Die nachstehende Tabelle 10 zeigt die Ergebnisse dieses Versuches.

**Tabelle 10: Einfluss des Alkaloidgehalts des Futtermittels auf die Futteraufnahme und das Wachstum bei Mastschweinen (Mittelwert ± Standardabweichung, n = 6)**

| | Kontrollgruppe | Versuchsgruppe |
|---|---|---|
| Ausgangsgewicht, kg | 35,8 ± 1,5 | 35,5 ± 0,8 |
| Endgewicht, kg | 43,8 ± 1,5 | 43,8 ± 1,0 |
| Futteraufnahme, g/Tag | 1779 ± 102 | 1834 ± 68 |
| tägliche Gewichtszunahme, g/Tag | 1008 ± 80 | 1047 ± 63 |
| Futterverwertung, g/g | 1,8 ± 0,1 | 1,8 ± 0,1 |

Obgleich die Futteraufnahme bei der Kontrollgruppe relativ hoch war, wurde sie bei der Versuchsgruppe durch α-Allocryptopin und die Benzophenänthridinalkaloide noch verbessert. Bei Fütterung ad libitum wurde bei der Versuchsgruppe eine um etwa 4 % höhere Futteraufnahme beobachtet. Dies wird auf eine höhere Tryptophanabsorption zurückgeführt. Aufgrund der Hemmung der Aromatische Aminosäure-Decarboxylase (AAD) durch die Benzophenanthridinalkaloide und die hepatoprotektive Wirkung des Alkaloids α-Allocryptopin steht einerseits mehr Tryptophan für eine erhöhte Produktion von Serotonin, das appetitanregend wirkt, zur Verfügung und andererseits werden durch die gesündere und leistungsfähigere Leber ebenfalls der Appetit und die Fresslust angeregt. Somit führt das erfindungsgemäße Futtermittel über den Schutz essentieller Aminosäuren und Nährstoffe zu einer verbesserten und preiswerteren Versorgung des Tieres mit essentiellen Nährstoffen, die ansonsten durch eine höhere Zumischung zum Futter unter deutlich höheren Kosten gegeben werden müssten, und verbessert überdies durch die hepatoprotektive Wirkung von α-Allocryptopin die Gesundheit des Tieres.

Bei der Versuchsgruppe wurde im Vergleich zur Kontrollgruppe eine Erhöhung der täglichen Gewichtszunahme beobachtet. Ein Einfluss des Alkaloidgehalts des Futtermittels auf die Futterverwertung wurde bei dieser Untersuchung nicht festgestellt, da das zusätzlich aufgenommene Futter für vermehrtes Wachstum genutzt wurde.

### Beispiel 9: Der Einfluss von α-Allocryptopin und Benzophenanthridinalkaloiden im Futtermittel auf den Tryptophan- und Lysin-Spiegel im Plasma

Dieses Beispiel untersucht den Einfluss von α-Allocryptopin und Benzophenanthridinalkaloiden auf den Plasmaspiegel der essentiellen Aminosäuren Tryptophan und Lysin bei Mastschweinen. Es soll gezeigt werden, dass Benzophenanthridinalkaloide, wie Sanguinarin und Chelerythrin, in Verbindung mit α-Allocryptopin essentielle Aminosäuren abbauende, unerwünschte bakterielle Enzyme irreversibel hemmen, womit mehr essentielle Nährstoffe wie essentielle Aminosäuren (Tryptophan, Lysin, Methionin) zur Absorption zur Verfügung stehen. Die Untersuchung soll zeigen, ob der verminderte unerwünschte Abbau essentieller Aminosäuren dann gemäß der Erfindung zu höheren Werten *in vivo* im Blut führt, die später für Wachstum und Leistung zur Verfügung stehen oder aber durch reduzierten Einsatz im Futter eingespart werden könnten.

12 kastrierte Schweine wurden getrennt in Stoffinrechselkäfigen gehalten. Die Kontrollgruppe und die Versuchsgruppe umfassten jeweils 6 Tiere. Die Zusammensetzung des Futters war für die Kontroll- und die Versuchsgruppe jeweils wie im Beispiel 8. Um einen direkten Vergleich der Ergebnisse zu ermöglichen, wurde restriktiv gefüttert, und zwar festgesetzt auf 95 g/kg metabolisches Körpergewicht (BW^{0,75}).

Die Adaptationsphase der Tiere betrug 10 Tage, die daran anschließende Versuchsphase 7 Tage. Am letzten Tag wurden vor der Fütterung und eine Stunde nach dem Füttern Blutproben für die Tryptophan- und Lysin-Analyse genommen.

Wie man anhand der Tabelle 11 sehen kann, zeigten die Versuche hinsichtlich der täglichen Gewichtszunahme und der Futterverwertung für die Versuchsgruppe bessere Ergebnisse als für die Kontrollgruppe.

**Tabelle 11: Effekt von α-Allocryptopin und Benzophenanthridinalkaloiden im Futtermittel auf die Wachstumsleistung von Mastschweinen (Mittelwert ± Standardabweichung, n = 6)**

| | Kontrollgruppe | Versuchsgruppe |
|---|---|---|
| Ausgangsgewicht, kg | 37,0 ± 0,6 | 36,5 ± 0,2 |
| Endgewicht, kg | 45,3 ± 0,9 | 44,9 ± 1,2 |
| tägliche Gewichtszunahme, g/Tag | 828 ± 25 | 841 ± 28 |
| Futterverwertung | 1,86 ± 0,06 | 1,84 ± 0,06 |

Die Untersuchung der prä- und der postprandialen Tryptophan- und Lysinkonzentration im Plasma zeigte für beide Gruppen einen signifikanten Anstieg. Die postprandiale Tryptophan- und Lysinkonzentration im Plasma war bei der Versuchsgruppe signifikant höher als bei der Kontrollgruppe. Dies zeigt, dass die Hemmung der Aminosäure-Decarboxylasen durch Benzophenanthridinalkaloide, insbesondere Sanguinarin, und α-Allocryptopin *in vivo* stattfindet, dass das "geschützte" Tryptophan und Lysin im Dünndarm aktiv zur Absorption zur Verfügung steht und dass das α-Allocryptopin und Benzophenanthridinalkaloide enthaltende Futtermittel eine bessere Nutzung von Tryptophan und Lysin im Futter ermöglicht.

### Beispiel 10: Einfluss von α-Allocryptopin und Benzophenanthridinalkaloiden auf laktierende Sauen und die Wurfleistung, hervorgerufen durch höheren Futterverzehr und höhere Nährstoffverfügbarkeit in vivo aufgrund der in den Beispielen 7 bis 9 gezeigten Effekte des erfindungsgemäßen Futtermittels.

Dieses Beispiel zeigt den Einfluss von α-Allocryptopin und Benzophenanthridinalkaloiden im Futter auf die Futteraufnahme durch laktierende Sauen und auf die Leistung der Sauen und ihrer Würfe während der Laktationszeit.

106 Sauen (72 gekreuzte Landrace x Large White, 34 Leicoma) in der ersten bis neunten Parität (3,6 ± 0,2, Mittelwert ± Standardabweichung) wurden entsprechend ihrer Parität untergebracht. Alle Tiere erhielten das gleiche Mais-Soja-Futter (metabolisierbare Energie 13,8 MJ/kg; 17,5 % XP), wobei diesem Futter im Falle der beiden Versuchsgruppen 30 bzw. 50 ppm des im Beispiel 8 genannten Additivs zugegeben wurden. Mit der Verabreichung dieses Futters wurde 4 Tage vor dem Abferkeln begonnen, und sie endete am Tag 20 (Absetzen) danach.

Hinsichtlich der Futteraufnahme wurde bei den Versuchsgruppen eine geringe Zunahme im Vergleich zur Kontrollgruppe beobachtet. Auch die Überlebensrate der Ferkel, gemessen am Tag 20, wurde durch den Alkaloidgehalt nicht beeinflusst. Beim Vergleich der Altsauen mit drei und mehr Geburten zeigten sich keine Unterschiede bei der Gewichtszunahme der Würfe.

Wenn man jedoch die Erstlingssauen und die Sauen mit der zweiten Geburt im Vergleich zwischen der Versuchsgruppe mit Benzophenanthridinalkaloiden und α-Allocryptopin vergleicht, zeigt sich ein vollkommen anderes Bild. Es zeigen sich signifikante Anstiege bei der Gewichtszunahme der Würfe der Versuchsgruppen im Vergleich zur Kontrollgruppe. Man kann also zu dem Schluss kommen, dass das Zusetzen von α-Allocryptopin und Benzophenanthridinalkaloiden zum Futter die Gewichtszunahme der Ferkel beim Wurf von Erstlingssauen während der Säugezeit positiv beeinflusst.

Aufgrund der Hemmung der AAD steht mehr Tryptophan zur Verfügung, das zu einem erhöhten Futterverzehr durch die Sauen führt. Dies in Verbindung mit mehr Lysin als wesentlichem Nährstoff für die Eiweißsynthese wiederum bewirkt eine erhöhte Milchproduktion, die sich ihrerseits positiv auf das Wachstum der Ferkel auswirkt.

### Beispiel 11: Einfluss von α-Allocryptopin und Benzophenanthridinalkaloiden im Futter auf die Leistungs- und Schlachtkörperparameter von Mastschweinen

Die Bestandteile des Futters (metabolisierbare Energie 13,8 MJ/kg; 1,00 % Lysin) waren:

| | |
|---|---|
| Gerste | 46 % |
| Weizen | 35,40 % |
| Sojaextraktionsschrot | 11,40 % |
| Fischmehl | 2,21 % |
| Sojaöl | 2,00% |
| Futterkalk | 1,00 % |
| Dicalciumphosphat | 0,74 % |
| Salz | 0,25 % |
| Prämix: Spurenelemente + Vitamine | 1,00 % |

Dem Futter der Versuchsgruppe wurden 30 ppm eines Additivs, bestehend aus Pflanzenmaterial der *Sanguinaria canadensis* und der *Macleaya cordata*, zugesetzt. Die darin enthaltenen Leitalkaloide sind hauptsächlich Sanguinarin und Chelerythrin sowie α-Allocryptopin. Alle Tiere wurden zweimal am Tag *semi ad libitum* gefüttert und hatten immer freien Zugang zu Wasser.

Das mittlere Körpergewicht am Beginn und am Ende der Versuche war wie folgt:

| | Kontrollgruppe | | Versuchsgruppe | |
|---|---|---|---|---|
| | Beginn | Ende | Beginn | Ende |
| Versuch | 30,1 ± 0,5 | 100,9 ± 7,4 | 30,3 ± 0,9 | 100,4 ± 5,4 |

In diesem Versuch zeigten sich hinsichtlich der täglichen Gewichtszunahme, der Futteraufnahme und der Futterverwertung keine Unterschiede zwischen der Kontrollgruppe und der Versuchsgruppe. Allerdings waren einige Schlachtkörper-Parameter der Versuchsgruppe deutlich besser als die der Kontrollgruppe, insbesondere wurde signifikant mehr Muskelfleisch sowie signifikant weniger Rückenspeckdicke festgestellt.

Die Ergebnisse des Versuchs sind in der Fig. 1 dargestellt. Dunkle Balken stellen die Ergebnisse für die Kontrollgruppe dar, dunkle schraffierte Balken jene der Versuchsgruppe. Die Figur 1 zeigt die signifikant positiven Ergebnisse hinsichtlich der Schlachtkörperqualität, wie signifikant verminderte Dicke des Rückenspecks und verbesserte Muskelfläche an der Rippe, die durch das alkaloidhaltige Futtermittel gemäß dieser Erfindung erzielt werden können.

Es hat sich gezeigt, dass ein Zusammenhang zwischen dem Magertleischansatz und dem verfügbaren Tryptophan und Lysin besteht, d.h. dass die Verwendung des alkaloidhaltigen Futtermittels durch Optimierung der Bilanz der essentiellen Aminosäuren zu einer verbesserten Proteinbilanz und in weiterer Folge zu mehr Magerfleisch und mehr Milcheiweiß führt.

## Patentansprüche

1. Futtermittel, welches herkömmliche Futterstoffe, wie Getreide oder Getreideprodukte, Mais, Proteine und essentielle Aminosäuren, Vitamine, mineralische Zuschlagstoffe, wie Salze, Phosphate, Kalk, Enzyme und dergleichen enthält, oder Futtermittelzusatz zur Herstellung derartiger Futtermittel, **dadurch gekennzeichnet, dass** das Futtermittel oder der Futtermittelzusatz ein Protopinalkaloid, insbesondere α-Allocryptopin, in wirksamer Menge als Leistungs- und Appetitförderer für Nutztiere enthält.

2. Futtermittel oder Futtermittelzusatz nach Anspruch 1, **dadurch gekennzeichnet, dass** es/er ein Protopinalkaloid, insbesondere α-Allocryptopin, in Kombination mit mindestens einem Benzophenanthridinalkaloid enthält.

3. Futtermittel oder Futtermittelzusatz nach einem der vorhergehenden Ansprüche, **dadurch gekennzeichnet, dass** α-Allocryptopin und/oder die Benzophenanthridinalkaloide in Form von Pflanzenmaterial oder als Pflanzenpresssaft zum Einsatz kommen.

4. Futtermittel oder Futtermittelzusatz nach einem der vorhergehenden Ansprüche, **dadurch gekennzeichnet, dass** α-Allocryptopin und/oder die Benzophenanthridinalkaloide in Form von Extrakten aus Pflanzenmaterial zum Einsatz kommen.

5. Futtermittel oder Futtermittelzusatz nach einem der vorhergehenden Ansprüche, **dadurch gekennzeichnet, dass** α-Allocryptopin in Form von Ptlanzenmaterial der *Macleya cordata* oder als Extrakt davon verwendet wird.

6. Futtermittel oder Futtermittetzusatz nach einem der vorhergehenden Ansprüche, **dadurch gekennzeichnet, dass** α-Allocryptopin und/oder die Benzophenanthridinalkaloide in Form isolierter Alkaloide oder Alkaloidgemische und/oder in Form ihrer Derivate und/oder synthetischen Analoge zum Einsatz kommen.

7. Verwendung von Protopinalkaloiden, insbesondere α-Allocryptopin, oder deren Derivaten oder synthetischen Analogen, bevorzugt in Kombination mit Benzophenanthridinalkaloiden oder deren Derivaten oder synthetischen Analogen, und/oder von diese Alkaloide enthaltendem Pflanzenmaterial bzw. Pflanzenpresssaft zur Leistungs- und Appetitförderung für Nutztiere.

8. Verwendung von Protopinalkaloiden, insbesondere α-Allocryptopin, oder deren Derivaten oder synthetischen Analogen, bevorzugt in Kombination mit Benzophenanthridinalkaloiden oder deren Derivaten oder synthetischen Analogen, und/oder von diese Alkaloide enthaltendem Pflanzenmaterial bzw. Pflanzenpresssaft zur Herstellung eines Futtermittels oder eines Futtermittelzusatzes mit hepatoprotektiver Wirkung für Nutz- und Hobbytiere.

9. Verwendung von Protopinalkaloiden, insbesondere α-Allocryptopin, zur Herstellung eines Arzneimittels gegen Lebererkrankungen, insbesondere Fettleber, bei Nutz- und Hobbytieren.

## Claims

1. Feed which comprises conventional feedstuffs such as cereals or cereal products, maize, proteins and essential amino acids, vitamins, mineral supplements, such as salts, phosphates, lime, enzymes and the like, or feed additive for producing such feeds, **characterized in that** the feed or the feed additive comprises a protopine alkaloid, in particular α-allocryptopine, in an amount active as performance promoter and appetite promoter for farm animals.

2. Feed or feed additive according to Claim 1, **characterized in that** it comprises a protopine alkaloid, in particular α-allocryptopine, in combination with at least one benzophenanthridine alkaloid.

3. Feed or feed additive according to one of the preceding claims, **characterized in that** α-allocryptopine and/or the benzophenanthridine alkaloids are used in the form of plant material or as plant press juice.

4. Feed or feed additive according to one of the preceding claims, **characterized in that** α-allocryptopine and/or the benzophenanthridine alkaloids are used in the form of extracts of plant material.

5. Feed or feed additive according to one of the preceding claims, **characterized in that** α-allocryptopine is used in the form of plant material of *Macleaya cordata* or as extract thereof.

6. Feed or feed additive according to one of the preceding claims, **characterized in that** α-allocryptopine and/or the benzophenanthridine alkaloids are used in the form of isolated alkaloids or alkaloid mixtures and/or in the form of their derivatives and/or synthetic analogues.

7. Use of protopine alkaloids, in particular α-allocryptopine, or their derivatives or synthetic analogues, preferably in combination with benzophenanthridine alkaloids or their derivatives or synthetic analogues, and/or of plant material or plant press juice comprising these alkaloids for performance promotion and appetite promotion for farm animals.

8. Use of protopine alkaloids, in particular α-allocryptopine, or their derivatives or synthetic analogues, preferably in combination with benzophenanthridine alkaloids or their derivatives or synthetic analogues, and/or of plant material or plant press juice comprising these alkaloids for producing a feed or a feed additive having hepatoprotective activity for farm animals and pets.

9. Use of protopine alkaloids, in particular α-allocryptopine, for producing a medicament for liver disorders, in particular fatty liver, in farm animals and pets.

## Revendications

1. Aliment pour animaux, contenant des matières nutritives habituelles, comme des céréales ou des produits issus de céréales, du maïs, des protéines et des acides aminés essentiels, des vitamines, des suppléments minéraux, comme des sels, des phosphates et de la chaux, des enzymes, et des substances similaires, ou adjuvant d'aliment conçu pour la préparation d'un tel aliment pour animaux, **caractérisé en ce que** l'aliment pour animaux ou l'additif d'aliment pour animaux contient un alcaloïde de la série de la protopine, en particulier de l'α-allocryptopine, en une quantité suffisante pour avoir pour effet d'améliorer l'appétit et d'améliorer les performances de production chez les animaux productifs.

2. Aliment pour animaux ou adjuvant d'aliment pour animaux, conforme à la revendication 1, **caractérisé en ce qu'**il contient un alcaloïde de la série de la protopine, en particulier de l'α-alloexyptopine, en association avec au moins un alcaloïde de la série de la benzophénanthridine.

3. Aliment pour animaux ou adjuvant d'aliment pour animaux, conforme à l'une des revendications précédentes, **caractérisé en ce qu'**on utilise l'α-allocryptopine et/ou les alcaloïdes de la série de la benzophénanthridine sous forme de matière végétale ou de jus de plantes pressées.

4. Aliment pour animaux ou adjuvant d'aliment pour animaux, conforme à l'une des revendications précédentes, **caractérisé en ce qu'**on utilise l'α-allocryptopine et/ou les alcaloïdes de la série de la benzophénanthridine sous forme d'extraits de matières végétales.

5. Aliment pour animaux ou adjuvant d'aliment pour animaux, conforme à l'une des revendications précédentes, **caractérisé en ce qu'**on utilise l'α-allocryptopine sous forme de matière végétale de *Macleya cordata* ou d'extrait de cette plante.

6. Aliment pour animaux ou adjuvant d'aliment pour animaux, conforme à l'une des revendications précédentes, **caractérisé en ce qu'**on utilise l'α-allocryptopine et/ou les alcaloïdes de la série de la benzophénanthridine sous forme d'alcaloïdes isolés ou de mélanges d'alcaloïdes et/ou sous forme de leurs dérivés et/ou de leurs analogues synthétiques.

7. Emploi d'alcaloïdes de la série de la protopine, en particulier de l'α-alloeryptopine, ou de dérivés ou d'analogues synthétiques de ces alcaloïdes, de préférence en association avec des alcaloïdes de la série de la benzophénanthridine ou des dérivés ou des analogues synthétiques de ces alcaloïdes, et/ou de matières végétales ou de jus de plantes pressées contenant de tels alcaloïdes, dans le but d'améliorer l'appétit et d'améliorer les performances de production chez les animaux productifs.

8. Emploi d'alcaloïdes de la série de la protopine, en particulier de l'α-allocryptopine, ou de dérivés ou d'analogues synthétiques de ces alcaloïdes, de préférence en association avec des alcaloïdes de la série de la benzophénanthridine ou des dérivés ou des analogues synthétiques de ces alcaloïdes, et/ou de matières végétales ou de jus de plantes pressées contenant de tels alcaloïdes, dans le but de fabriquer un aliment pour animaux ou un adjuvant d'aliment pour animaux qui exerce un effet de protection du foie chez les animaux productifs et les animaux de compagnie.

9. Emploi d'alcaloïdes de la série de la protopine, en particulier de l'α-allocxyptopine, dans le but de fabriquer un médicament contre des maladies du foie, en particulier la stéatose hépatique, chez les animaux productifs et les animaux de compagnie.
